# EUROPÄISCHE PATENTSCHRIFT

(11) **EP 1 814 469 B1**
(45) Veröffentlichungstag und Bekanntmachung des Hinweises auf die Patenterteilung: **23.03.2011**
(21) Anmeldenummer: 05823662.1
(22) Anmeldetag: 18.11.2005
(51) Int. Cl.: A61B 17/32

(54) **VORRICHTUNG ZUR MUKOSARESEKTION**
DEVICE FOR MUCOSA RESECTION
DISPOSITIF DE RESECTION MUQUEUSE

(30) Priorität: 27.11.2004 DE 102004057366
(43) Veröffentlichungstag der Anmeldung: 08.08.2007
(73) Patentinhaber: Erbe Elektromedizin GmbH, 72072 Tübingen (DE)
(72) Erfinder: FISCHER, Klaus, 72202 Nagold (DE)
(74) Vertreter: Bohnenberger, Johannes
(86) Internationale Anmeldenummer: PCT/EP2005/012391
(87) Internationale Veröffentlichungsnummer: WO 2006/056371

(56) Entgegenhaltungen:
- EP-A- 0 551 920
- US-A- 5 871 462
- US-A1- 2002 177 802
- US-B1- 6 322 533

## Beschreibung

Die Erfindung betrifft eine Vorrichtung zur Mukosaresektion, insbesondere mittels endoskopischer Methoden.

Zur Behandlung gastrointestinaler Karzinome müssen Mukosaresektionen vorgenommen werden, die üblicherweise mittels endoskopischer Methoden durchgeführt werden. Veränderungen des Gewebes werden nach dem Erkennen markiert (gefärbt) und dann mit physiologischer Kochsalzlösung unterspritzt. Dadurch wird die Mukosa von der darunter liegenden Schicht, der Muskularis, abgehoben. Dadurch können ein ausreichender Sicherheitsabstand zur Muskularis erreicht und damit eine mögliche Perforation vermieden werden. Das Abtragen der geschädigten Mukosa kann mittels verschiedenster Techniken geschehen, z.B. durch Schlingen oder durch Nadelmesser. In allen Fällen aber ist es äußerst wünschenswert, eine gezielte, räumlich begrenzte Abhebung der Mukosa und eine entsprechend gezielte und räumlich begrenzte Abtrennung zu gewährleisten, was üblicherweise mit einem erheblichen Operationsaufwand verbunden ist, der eine große Geschicklichkeit und Erfahrung des Operateurs erfordert.

Insbesondere bereitet es erhebliche Probleme, zunächst mit einer (flexiblen) Nadel in die Submukosa einzustechen und eine gewünschte (kleine) Flüssigkeitsmenge zum Abheben der Mukosa zu injizieren. Die Einstichtiefe, die Dosierung und damit die Ausbildung der Abhebung sind vom OP-Personal abhängig. Nach dem Abheben der Mukosa muß deren Abtrennung erfolgen, wobei die Zeit nach dem Einspritzen und Abheben der Mukosa eine wesentliche Rolle spielt, da die eingespritzte Flüssigkeit aus der Submukosa entweicht und die Abhebung somit zurückgeht, bis keine Abtrennung des geschädigten Gewebes mehr erfolgen kann. In solchen Fällen muß erneut unterspritzt und versucht werden, die gewünschte Abtragung vorzunehmen. Besonders dann, wenn ein Tumor hinter einer Falte liegt, kann eine Schlinge nicht schnell genug um die Läsion gelegt oder fixiert werden.

Aus der US-A-5 871 462, US-B1-6 322 533, EP-A-0 551 920 und US 2002/177802 A1 sind Vorrichtungen bekannt, mittels derer Wasserstrahlen bei verschiedenen Drücken erzeugbar sind. Insbesondere wird in der US-A-5 871 462 vorgeschlagen, zu Beginn einer Operation den Flüssigkeitsstrahl mit einem niedrigen Druck austreten zu lassen, um so zu zielen, wonach dann auf einen höheren Druck zum Schneiden umgeschaltet wird. In der US-B-1-6 322 533 wird vorgeschlagen, ein und dasselbe Instrument zum Schneiden und zum Spülen, also bei einem sehr viel niedrigeren Druck zu verwenden. Die besondere Problematik einer Mukosaresektion wird in den Druckschriften nicht angesprochen.

Der Erfindung liegt die Aufgabe zugrunde, eine Vorrichtung zur Mukosaresektion aufzuzeigen, mittels derer in vereinfachter Weise Mukosagewebe sicher entfernt werden kann.

Diese Aufgabe wird durch eine Vorrichtung nach Patentanspruch 1 gelöst.

Insbesondere wird die Aufgabe durch eine Vorrichtung zur endoskopischen Mukosaresektion gelöst, umfassend
eine Einrichtung zur endoskopischen Wasserstrahlchirurgie mit einer Regeleinrichtung zum Regeln eines Druckes, mit dem ein Wasserstrahl aus einer Düse zur Erzielung einer vorbestimmten Energie ausgestoßen wird, und mit einer Einstellvorrichtung zum Einstellen des Druckes auf einen ersten Wert, bei welchem der Wasserstrahl in die Mukosa so eindringt, daß diese durch Bildung eines Flüssigkeitsdepots von der darunter liegenden Muskularis abgehoben wird und auf einen zweiten, vom ersten Druck unterschiedlichen Druck, bei dem die Mukosa durch den Wasserstrahl schnittförmig durchtrennbar ist.

Es wird also mittels eines einzigen Instrumentes sowohl die Unterspritzung zum Abheben der Mukosa bewerkstelligt als auch das Durchtrennen des Gewebes, und zwar lediglich durch eine geeignete Einstellung des Druckes.

Ein weiterer Vorteil der erfindungsgemäßen Vorrichtung liegt auch darin, daß durch das Durchtrennen zumindest Teile der das Trennen bewerkstelligenden Flüssigkeit aus dem Depot austretende Flüssigkeit ersetzt und so die Abhebung erhält.

Vorzugsweise ist die Einstellvorrichtung derart ausgebildet, daß bei Einstellen des Druckes auf den ersten Wert und Betätigen eines ersten Startschalters eine vorbestimmte Flüssigkeitsmenge ausgestoßen wird. Dadurch kann eine sehr einfache und genaue Dosierung der Flüssigkeitsmenge erfolgen, welche die Mukosa abhebt.

Die Einstellvorrichtung ist weiterhin vorzugsweise derart ausgebildet, daß bei Einstellen des Druckes auf den zweiten Wert und bei Betätigen eines zweiten Startschalters die Flüssigkeit während des Betätigens des zweiten Startschalters im wesentlichen kontinuierlich ausgestoßen wird. Dadurch wird das Durchtrennen des Gewebes ganz in die Hand des Operateurs gegeben.

Die Einstellvorrichtung ist weiterhin vorzugsweise derart ausgebildet, daß der erste Druck unabhängig vom zweiten Druck einstellbar ist. Dadurch kann der zum Abheben der Mukosa und der zu ihrem Durchtrennen notwendige Druck jeweils den physiologischen Gegebenheiten angepaßt werden.

Es ist auch möglich, die Einstellvorrichtung mit einem Verhältnis-Einstellorgan auszustatten, welches es ermöglicht, ein vorbestimmtes Verhältnis des ersten zum zweiten Druck einzustellen. Insbesondere dann, wenn ein Spitzendruck-Einstellorgan vorgesehen ist, kann so ein schnelleres Anpassen auf sich ändernde Gewebeverhältnisse vorgenommen werden.

Der zweite Druck ist vorzugsweise in mehreren Stufen zur Anpassung an verschiedene Gewebearten einstellbar, so daß eine leichte Bedienbarkeit durch Auswahl weniger Werte gewährleistet ist. Die Drücke in den verschiedenen Stufen sind vorzugsweise unabhängig voneinander einstellbar, was wiederum die Handhabung während einer Operation erleichtert.

Vorzugsweise ist ein Anzeigeorgan vorgesehen zur Anzeige von verschiedenen Betriebsmodi, insbesondere in Bezug auf einen momentan eingestellten Druckwert oder auch einen zuvor eingestellten Druckwert zum Abheben oder zum Durchtrennen der Mukosa, so daß der Operateur leicht sehen kann, mit welchen Werten er bisher arbeitete bzw. welche Werte momentan eingestellt sind.

Zur Bedienung der Vorrichtung ist vorzugsweise ein Fußschalter vorgesehen zum wahlweisen einstellen des ersten oder des zweiten Druckes, also zum Umschalten zwischen Unterspritzen und Durchtrennen. In diesem Fall ist es auch möglich, mit einem einzigen Startschalter zu arbeiten, der den jeweiligen Vorgang (Unterspritzen/Trennen) auslöst.

Bevorzugte Ausführungsformen der Erfindung ergeben sich aus den Unteransprüchen.

Nachfolgend wird ein Ausführungsbeispiel der Erfindung anhand einer Abbildung erläutert, welche in schematisierter Darstellung die erfindungsgemäße Vorrichtung zeigt.

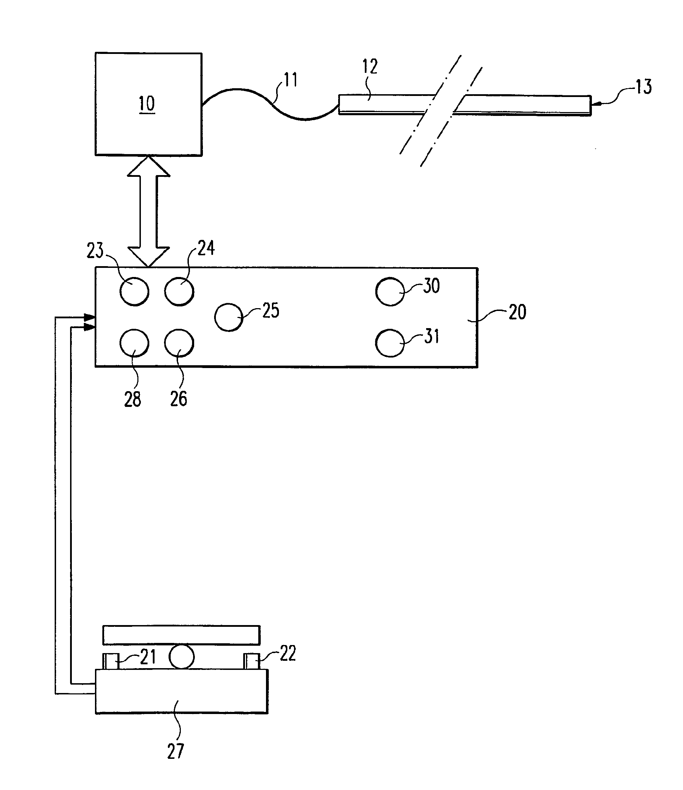

Wie in der Abbildung dargestellt, ist eine Wasserstrahl-Einrichtung 10 vorgesehen, wie diese an sich bekannt ist. Über eine Zuführungsleitung 11 wird Wasser (physiologische Kochsalzlösung) einer Sonde 12 zugeführt, die in den Arbeitskanal eines Endoskops einführbar ist. Der Wasserstrahl tritt aus einer Düse 13 am distalen Ende der Sonde 12 aus. Zur Steuerung der Wasserstrahleinrichtung 10 ist eine Einstellvorrichtung 20 vorgesehen, die mit verschiedenen Einstellorganen ausgestattet ist. Insbesondere sind hier ein erstes Druckeinstellorgan 23 und ein zweites Druckeinstellorgan 24 vorgesehen, zum Vorwählen des ersten bzw. des zweiten Druckes. Über ein Einstellorgan 25 kann das Verhältnis des ersten zum zweiten Druck eingestellt werden und über ein Organ 26 ist der Spitzendruck einstellbar. Diese Druckeinstellungen sind nicht alle voneinander unabhängig, so daß eine Einstellung entweder durch die Einstellorgane 23 und 24 oder durch die Einstellung der Organe 25, 26 erfolgt.

In beiden Fällen wird die Spritzdauer bei Applikation des ersten Druckes über ein Einstellorgan 28 eingestellt.

Zur Betätigung ist ein Fußschalter 27 vorgesehen, der einen ersten Startschalter 21 und einen zweiten Startschalter 22 aufweist. Je nachdem, welcher der beiden Startschalter 21 bzw. 22 betätigt wird, arbeitet die Vorrichtung mit dem ersten oder dem zweiten Druck. Es ist natürlich auch möglich, den Fußschalter 27 derart auszubilden, daß er lediglich den Druck wählt, während das Startsignal durch einen anderen Schalter, z.B. einen handbetätigten Schalter, gegeben wird. Es ist ebenfalls möglich, statt eines Fußschalters entsprechende Handschalter vorzusehen.

Schließlich weist die Einstellvorrichtung 20 noch Anzeigeorgane 30 und 31 zum Anzeigen des ersten bzw. zweiten Druckes auf, wobei diese Anzeigeorgane derart ausgebildet sind, daß eine schnelle Kontrolle zum Feststellen, welcher der beiden Drücke eingestellt ist, ebenso ermöglicht wird, wie eine exakte Kontrolle des eingestellten Druckes (als Meßwert).

Bei einer alternativen, in den Abbildungen nicht gezeigten Ausführungsform der Erfindung ist es vorgesehen, den Wasserstrahl mit dem ersten Druck aus einer anderen Düse 13 austreten zu lassen als den Wasserstrahl mit dem zweiten Druck. Dadurch ist es möglich, nicht nur den Strahldurchmesser, sondern auch die Strahlrichtung unterschiedlich zu wählen, je nachdem, ob Gewebe unterspritzt oder getrennt werden soll.

### Bezugszeichenliste

- 10: Wasserstrahleinrichtung
- 11: Zuführung
- 12: Sonde
- 13: Düse
- 20: Einstellvorrichtung
- 21: erster Startschalter
- 22: zweiter Startschalter
- 23: erstes Druckeinstellorgan
- 24: zweites Druckeinstellorgan
- 25: Verhältniseinstellorgan
- 26: Spitzendruckeinstellorgan
- 27: Fußschalter
- 28: Spritzdauereinstellorgan
- 30: erstes Anzeigeorgan (erster Druck)
- 31: zweites Anzeigeorgan (zweiter Druck)

## Patentansprüche

1. Chirurgische Vorrichtung, umfassend eine Einrichtung (10) zur endoskopischen Wasserstrahlchirurgie
mit einer Regeleinrichtung zum Regeln eines Druckes, mit dem ein Wasserstrahl aus einer Düse (13) zur Erzielung einer vorbestimmten Energie ausgestoßen wird, und
mit einer Einstellvorrichtung (20) zum Einstellen eines Druckes auf einen ersten und einen zweiten Wert,
**dadurch gekennzeichnet, daß**
die Einstellvorrichtung (20) derart auf einen ersten Wert einstellbar ist, dass bei diesem Wert der Wasserstrahl in die Mukosa so eindringt, dass diese zur Bildung eines Flüssigkeitsdepots von der darunter liegenden Muskularis abgehoben wird, und
auf einen zweiten Druck einstellbar ist, der vom ersten Druck unterschiedlich ist und bei dem die Mukosa durch den Wasserstrahl schnittförmig durchtrennbar ist,
wobei die Einstellvorrichtung (20) weiterhin derart ausgebildet ist, dass bei Einstellen des Druckes auf den ersten Wert und Betätigen eines ersten Startschalters (21) eine vorbestimmte Flüssigkeitsmenge ausgestoßen wird.

2. Chirurgische Vorrichtung nach einem der vorhergehenden Ansprüche,
**dadurch gekennzeichnet, daß**
die Einstellvorrichtung (20) derart ausgebildet ist, daß bei Einstellen des Druckes auf den zweiten Wert und Betätigen eines zweiten Startschalters (22) die Flüssigkeit während des Betätigens des zweiten Startschalters (22) im wesentlichen kontinuierlich ausgestoßen wird.

3. Chirurgische Vorrichtung nach einem der vorhergehenden Ansprüche,
**dadurch gekennzeichnet, daß**
die Einstellvorrichtung (20) derart ausgebildet ist, daß der erste Druck unabhängig vom zweiten Druck einstellbar ist.

4. Chirurgische Vorrichtung nach einem der Ansprüche 1 bis 2,
**dadurch gekennzeichnet, daß**
die Einstellvorrichtung ein Verhältnis-Einstellorgan (25) aufweist zur Einstellung eines vorbestimmten Verhältnisses des ersten Druckes zum zweiten Druck.

5. Chirurgische Vorrichtung nach Anspruch 4,
**dadurch gekennzeichnet, daß**
ein Spitzendruck-Einstellorgan (26) vorgesehen ist zum Einstellen eines Maximaldruckes, mit dem der Wasserstrahl aus der Düse (13) herausgedrückt wird.

6. Chirurgische Vorrichtung nach einem der vorhergehenden Ansprüche,
**dadurch gekennzeichnet, daß**
der zweite Druck in mehreren Stufen zur Anpassung an verschiedene Gewebearten einstellbar ist.

7. Chirurgische Vorrichtung nach Anspruch 6,
**dadurch gekennzeichnet, daß**
die Drücke in den Stufen voneinander unabhängig einstellbar sind.

8. Chirurgische Vorrichtung nach einem der vorhergehenden Ansprüche,
**gekennzeichnet durch**
ein Anzeigeorgan (30, 31) zur Anzeige von verschiedenen Betriebsmodi, insbesondere in Bezug auf einen momentan oder zuvor eingestellten Druckwert zum Abheben oder zum Durchtrennen der Mukosa.

9. Chirurgische Vorrichtung nach einem der vorhergehenden Ansprüche,
**dadurch gekennzeichnet, daß**
die Einstellvorrichtung (20) einen Fußschalter (27) zum wahlweisen Einstellen des ersten oder zweiten Druckes aufweist.

## Claims

1. Surgical apparatus comprising a device (10) for endoscopic water-jet surgery,
having a control device for controlling the pressure with which a water jet is expelled from a nozzle (13) in order to attain a predetermined energy, and
having a setting apparatus (20) for setting the pressure to a first value and a second value,
**characterised in that**
the setting apparatus (20) can be set to a first value so that, at that value, the water jet so penetrates the mucosa that the latter is lifted off from the underlying muscularis for formation of a pool of liquid and
can be set to a second pressure, which is different from the first pressure and at which the mucosa can be sliced through in the form of a cut by means of the water jet,
the setting apparatus (20) moreover being so constructed that, on setting the pressure to the first value and actuating a first start switch (21), a predetermined amount of liquid is expelled.

2. Surgical apparatus according to one of the preceding claims,
**characterised in that**
the setting apparatus (20) is so constructed that, on setting the pressure to the second value and actuating a second start switch (22), the liquid is expelled substantially continuously during actuation of the second start switch (22).

3. Surgical apparatus according to one of the preceding claims,
**characterised in that**
the setting apparatus (20) is so constructed that the first pressure can be set independently of the second pressure.

4. Surgical apparatus according to one of claims 1 to 2,
**characterised in that**
the setting apparatus has a ratio-setting means (25) for setting a predetermined ratio of the first pressure to the second pressure.

5. Surgical apparatus according to claim 4,
**characterised in that**
a peak-pressure-setting means (26) is provided for setting a maximum pressure at which the water jet is discharged from the nozzle (13).

6. Surgical apparatus according to one of the preceding claims,
**characterised in that**
the second pressure can be set at a plurality of levels for matching various types of tissue.

7. Surgical apparatus according to claim 6,
**characterised in that**
the pressures at the levels can be set independently of one another.

8. Surgical apparatus according to one of the preceding claims,
**characterised by**
a display means (30, 31) for displaying various modes of operation, especially in respect of a currently or previously set value of pressure for lifting off or slicing through the mucosa.

9. Surgical apparatus according to one of the preceding claims,
**characterised in that**
the setting apparatus (20) has a foot switch (27) for setting the first or second pressure as desired.

## Revendications

1. Dispositif chirurgical, comprenant un appareil (10) pour la chirurgie endoscopique à jet d'eau,
avec un dispositif de régulation pour régler une pression par laquelle un jet d'eau est éjecté d'une buse (13) pour obtenir une énergie prédéterminée, et
avec un dispositif d'ajustement (20) pour ajuster une pression sur une première et une deuxième valeur,
**caractérisé en ce que**
le dispositif d'ajustement (20) peut être ajusté à une valeur de telle sorte qu'à cette valeur, le jet d'eau pénètre dans la muqueuse de telle sorte que celle-ci soit décollée pour former un dépôt par liquide des muscles sous-jacents, et
qu'il peut être ajusté à une deuxième pression qui est différente de la première pression et à laquelle la muqueuse peut être séparée sous forme de coupure par le jet d'eau,
le dispositif d'ajustement (20) étant en outre conçu de telle sorte que lors de l'ajustement de la pression à la première valeur et de l'actionnement d'un premier commutateur de démarrage (21), une quantité prédéterminée de liquide soit éjectée.

2. Dispositif chirurgical selon la revendication précédente,
**caractérisé en ce que**
le dispositif d'ajustement (20) est conçu de telle sorte que lors de l'ajustement de la pression à la deuxième valeur et de l'actionnement d'un deuxième commutateur de démarrage (22), le liquide soit éjecté pendant l'actionnement du deuxième commutateur de démarrage (22) de façon essentiellement constante.

3. Dispositif chirurgical selon l'une des revendications précédentes,
**caractérisé en ce que**
le dispositif d'ajustement (20) est conçu de telle sorte que la première pression soit ajustable indépendamment de la deuxième pression.

4. Dispositif chirurgical selon l'une des revendications 1 à 2,
**caractérisé en ce que**
le dispositif d'ajustement présente un organe d'ajustement de rapport (25) pour ajuster un rapport prédéterminé de la première pression à la deuxième pression.

5. Dispositif chirurgical selon la revendication 4,
**caractérisé en ce**
**qu'**un organe d'ajustement de la pression de pointe (26) est prévu pour ajuster une pression maximale à laquelle le jet d'eau est éjecté de la buse (13).

6. Dispositif chirurgical selon l'une des revendications précédentes,
**caractérisé en ce**
**que** la deuxième pression est ajustable à plusieurs niveaux pour l'adapter à divers types de tissus.

7. Dispositif chirurgical selon la revendication 6,
**caractérisé en ce que**
les pressions sont ajustables indépendamment les unes des autres aux différents niveaux.

8. Dispositif chirurgical selon l'une des revendications précédentes,
**caractérisé par**
un organe d'affichage (30, 31) pour afficher différents modes de fonctionnement, en particulier par rapport à une valeur de pression momentanée ou ajustée au préalable pour décoller ou séparer la muqueuse.

9. Dispositif chirurgical selon l'une des revendications précédentes,
**caractérisé en ce que**
le dispositif d'ajustement (20) présente un commutateur à pied (27) pour ajuster au choix la première ou deuxième pression.
